# EUROPEAN PATENT APPLICATION

(11) **EP 3 354 645 A1**
(43) Date of publication of application: **01.08.2018**
(21) Application number: 17153423.3
(22) Date of filing: 26.01.2017
(51) Int. Cl.: C07D 311/80, C07C 67/00, C07C 69/76, C07C 51/00, C07C 63/00

(54) **PROCESS FOR PREPARING UROLITHINS**

(71) Applicant: Patheon Austria GmbH & Co KG, Linz (AT)
(72) Inventor: The designation of the inventor has not yet been filed

(57) **Abstract**

The invention pertains to a process for preparing a compound of formula (1) 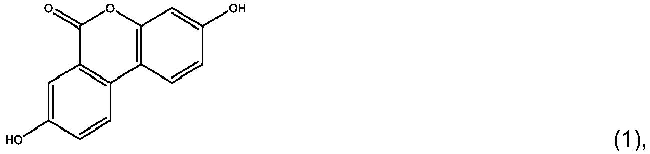 which process comprises the steps of contacting the compound of formula (5) 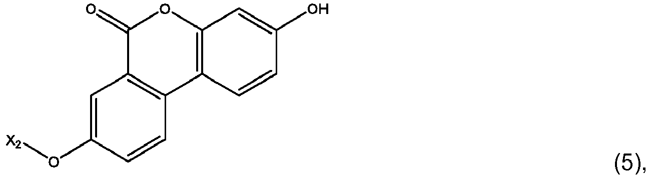 wherein X₂ is hydrogen or a protecting group selected from isopropyl, tert-butyl and a protecting group comprising an oxygen;
with an acid to give the compound of formula (1).

## Description

The present invention relates to processes for preparing urolithins.

Synthetic routes for the preparation of urolithins are known in the art. For example, WO 2014/004902 and WO 2012/088519 disclose the preparation of urolithin A (see Figure below).

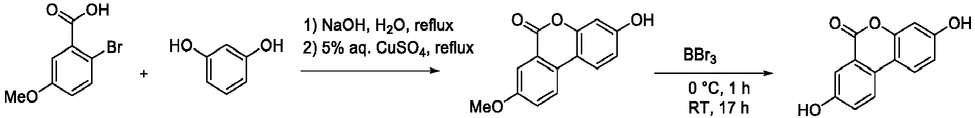

In this process the conversion of compound 3 to compound 4 is generally incomplete, resulting in a low overall yield of urolithin A. Moreover, the use of BBr₃ is undesirable due to its hazardous properties.

The objective of the present invention is to provide a novel process as well as an improved process to prepare urolithins.

The present invention pertains to a process for preparing a compound of formula (1) 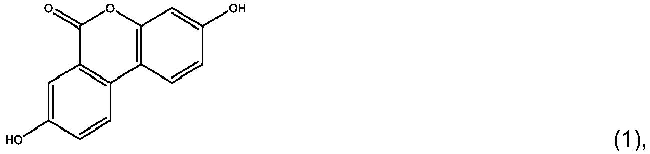 which process comprises the steps of:
a) contacting a compound of formula (2) 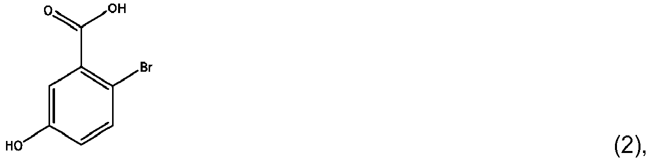 with protecting group precursor in a solvent, and converting the compound of formula (2) into a compound of formula (3) 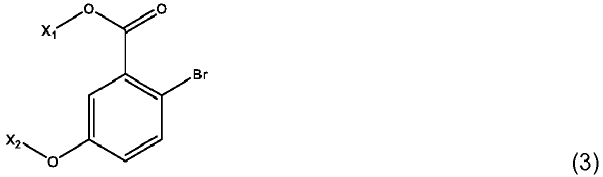
b) followed by contacting the compound of formula (3) with a base to give a compound of formula (4) 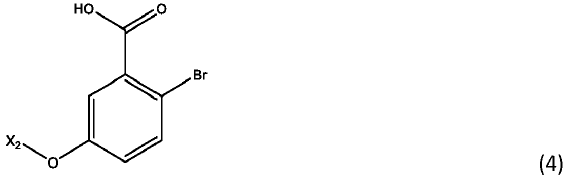
c) followed by contacting the compound of formula (4) with resorcinol in the presence of a base to give a compound of formula (5) 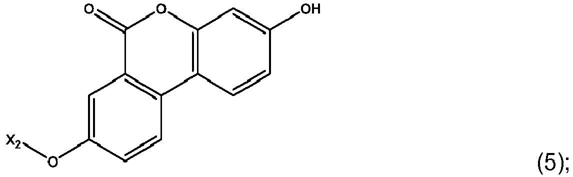 and
d) followed by contacting the compound of formula (5) with an acid to give the compound of formula (1),
   and wherein at least one of the protecting groups X₁ and X₂ is hydrogen or a protecting group selected from isopropyl, tert-butyl and a protecting group comprising an oxygen.

The protecting groups X₁ and X₂, which at least one of the protecting groups X₁ and X₂ is hydrogen or a protecting group selected from isopropyl, tert-butyl and a protecting group comprising an oxygen, can be any suitable protecting groups. X₁ and X₂ can be the same or different. Preferably, X₁ and X₂ are the same. Preferably, at least one of the protecting groups X₁ and X₂ comprises oxygen. The protecting groups X₁ and X₂ can be an -R₁-O-R₂, wherein R₁ and R₂ are independently C₁ to C₄ alkyl and wherein R₁ and R₂ can form a cyclic structure comprising 4, 5 or 6 carbon atoms. Examples of X₁ and X₂ include alkoxy alkyl such as methoxy methyl, ethoxy methyl, methoxy ethyl and ethoxy ethyl; tetrahydropyranyl (THP), ethyl vinyl ether and tert-butyl. Preferably, X₁ and X₂ are alkoxy alkyl. More preferably, X₁ and X₂ are selected from methoxy methyl and ethoxy methyl. Most preferably, X₁ and X₂ are methoxy methyl.
It is also contemplated to use two different protecting groups X₁ and X₂, and wherein at least one of the protecting groups X₁ and X₂ is hydrogen or a protecting group selected from isopropyl, tert-butyl and a protecting group comprising an oxygen. The other protecting group can be any protecting group known in the art and hydrogen; such protecting groups include alkyls such as methyl, ethyl, isopropyl and tert-butyl; benzyl; and protecting groups comprising oxygen. In one embodiment, X₁ can be an alkyl group, including methyl, ethyl and propyl, and X₂ can be an alkoxy alkyl group, including methoxy methyl and ethoxy methyl. The protecting group X₂ preferably is not methyl group as this metyl protecting group is not easy to cleave. The protecting groups X₁ and X₂ are introduced in step (a) of the process using protecting group precursors. Examples of such protecting group precursors include halogenated protecting groups, preferably chlorinated protecting groups, including methoxy methyl chloride and ethoxy methyl chloride.

The process of the invention allows for the preparation of the compound of formula (1) with a good yield and good selectivity. In the process of the invention novel intermediates were used. A further advantage is that the protecting groups can be easily cleaved. Also the use of hazardous reagents like BBr₃ and AlCl₃ is avoided. Consequently, the inventive process is suitable for commercial scale production of urolithins.

The process of the invention is further explained below when describing the details of the separate steps. These details also apply to the process of the invention.

The invention pertains to a process for preparing a compound of formula (1) 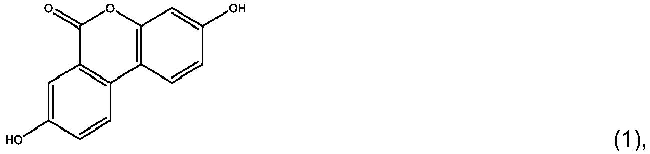 which process comprises the steps of contacting the compound of formula (5) 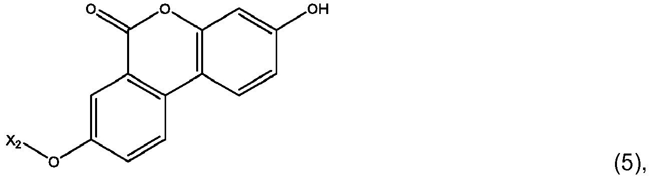 wherein X₂ is hydrogen or a protecting group selected from isopropyl, tert-butyl and a protecting group comprising an oxygen;
with an acid to give the compound of formula (1).
The protecting group X₂ is as defined above.

The compound of formula (5) is contacted with an acid capable of removing the X₂ protecting group. This process step is similar to step (d) in the overall process of the invention. The acid may be any acid that can be suitably used in the process of the present invention. Examples of such acids include sulfuric acid, hydrogen chloride, *p*-toluene sulfonic acid (pTsOH) and methane sulfonic acid. Preferably, the acid is sulfuric acid.

In one embodiment of the invention, the molar ratio of the acid and the compound of formula (5) is at least 0.001, preferably at least 0.003, more preferably at least 0.005 and most preferably at least 0.01, and generally at most 1, preferably at most 0.5, more preferably at most 0.3 and most preferably at most 0.2.
In one aspect, the acid may be present as solvent or co-solvent.

Optionally, a solvent is present during this step of the process. Examples of suitable solvents include alcohols such as methanol, ethanol or isopropanol; alkylene glycols such as ethylene glycol and propylene glycol; water and weak acid in water, e.g. acetic acid in water. Also a combination of two or more solvents is contemplated. Preferably, the solvent is an alcohol, in particular methanol, ethanol and isopropanol. Most preferably, the solvent is methanol.

On one embodiment, the reaction mixture comprises the solvent in an amount of at least 50 % by weight (wt%), based on the total weight of the reaction mixture. Preferably, the solvent is present in an amount of at least 55 wt%, more preferably at least 60 wt%, even more preferably at least 65 wt% and most preferably at least 70 wt%, and preferably at most 99 wt%, more preferably at most 95 wt%, even more preferably at most 90 wt% and most preferably at most 85 wt%, based on the total weight of the reaction mixture. The reaction mixture includes all ingredients that are necessary to convert the compound of formula (5) into the compound of formula (1), i.e. it includes the compound of formula (5), the acid, the (optional) solvent and any other ingredient present.

In one aspect, this process step is conducted at a temperature of at least 20°C, preferably at least 25°C and most preferably at least 30°C, and preferably at most 100°C, more preferably at most 90°C and most preferably at most 80°C.

The invention further pertains to a process for preparing a compound of formula (5) 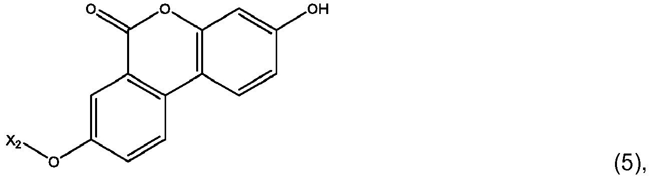 wherein X₂ is a protecting group selected from a protecting group comprising an oxygen, isopropyl, tert-butyl and hydrogen; which process comprises the steps of contacting the compound of formula (4) 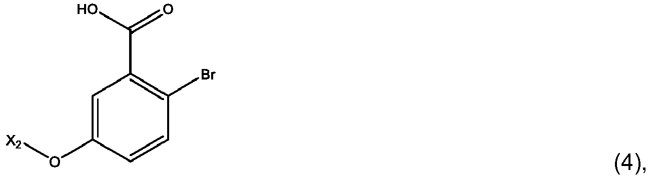 wherein X₂ is hydrogen or a protecting group selected from isopropyl, tert-butyl and a protecting group comprising an oxygen,
with resorcinol in the presence of a base to give the compound of formula (5).
The protecting group X₂ is as defined above.

The compound of formula (4) is contacted with resorcinol in the presence of a base. This process step is similar to step (c) in the overall process of the invention. The base can be any base that can be suitably used in the process of the present invention. Examples of such bases include sodium hydroxide, potassium hydroxide, sodium carbonate, cesium carbonate and diisopropyl ethyl amine. Preferably, the base is selected from sodium hydroxide and potassium hydroxide. Most preferably, the base is sodium hydroxide.

In one embodiment of the invention, the molar ratio of the base and the compound of formula (4) is at least 0.1, preferably at least 0.5, more preferably at least 0.8 and most preferably at least 1, and generally at most 10, preferably at most 8, more preferably at most 6 and most preferably at most 5.

In one embodiment of the invention, the molar ratio of resorcinol and the compound of formula (4) is at least 0.1, preferably at least 0.5, more preferably at least 0.8 and most preferably at least 1, and generally at most 10, preferably at most 8, more preferably at most 6 and most preferably at most 5.

Optionally, a solvent is present during this process step. Examples of suitable solvents include alcohols such as methanol, ethanol and isopropanol; alkylene glycols such as ethylene glycol, propylene glycol; and water. Also a combination of two or more solvents is contemplated. Preferably, the solvent is selected from alcohol, in particular methanol, ethanol and isopropanol, and water. Most preferably, the solvent is water.

In one embodiment, the reaction mixture comprises the solvent in an amount of at least 50 % by weight (wt%), based on the total weight of the reaction mixture. Preferably, the solvent is present in an amount of at least 55 wt%, more preferably at least 60 wt%, even more preferably at least 65 wt% and most preferably at least 70 wt%, and preferably at most 99 wt%, more preferably at most 95 wt%, even more preferably at most 90 wt% and most preferably at most 85 wt%, based on the total weight of the reaction mixture. The reaction mixture includes all ingredients that are necessary to convert the compound of formula (4) into the compound of formula (5), i.e. it includes the compound of formula (4), resorcinol, the base, the (optional) solvent and any other ingredient present.

Optionally, a catalyst is present during this process step. Examples of suitable catalysts include salts of Cu(I) such as copper(I) iodide (CuI) and salts of Cu(II) such as copper(II) sulfate (CuSO₄). Of these catalysts copper(II) sulfate is preferred.

In one embodiment of the invention, the molar ratio of the catalyst and the compound of formula (4) is at least 0.001, preferably at least 0.002, more preferably at least 0.005 and most preferably at least 0.01, and generally at most 1, preferably at most 0.5, more preferably at most 0.2 and most preferably at most 0.1.

In one aspect, this process step is conducted at a temperature of at least 50°C, preferably at least 55°C and most preferably at least 60°C, and preferably at most 100°C, more preferably at most 90°C and most preferably at most 80°C.

The invention further pertains to a process for preparing a compound of formula (4) 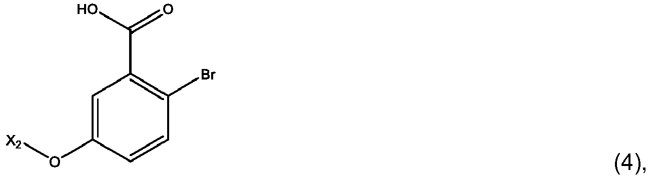 wherein X₂ is hydrogen or a protecting group selected from isopropyl, tert-butyl and a protecting group comprising an oxygen,
which process comprises the steps of contacting the compound of formula (3) 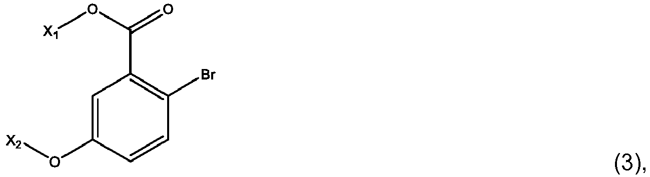 wherein X₁ and X₂ are protecting groups, and wherein at least one of the protecting groups X₁ and X₂ is hydrogen or a protecting group selected from isopropyl, tert-butyl and a protecting group comprising an oxygen,
with a base to give the compound of formula (4).
The protecting groups X₁ and X₂ are as defined above.

The compound of formula (3) is contacted with a base. This process step is similar to step (b) in the overall process of the invention. The base can be any base that can be suitably used in the process of the present invention. Examples of such bases include sodium hydroxide, potassium hydroxide, sodium carbonate and cesium carbonate. Preferably, the base is selected from sodium hydroxide and potassium hydroxide. Most preferably, the base is sodium hydroxide.

In one embodiment of the invention, the molar ratio of the base and the compound of formula (4) is at least 0.1, preferably at least 0.5, more preferably at least 1 and most preferably at least 2, and generally at most 10, preferably at most 8, more preferably at most 6 and most preferably at most 5.

Optionally, a solvent is present during this process step. Examples of suitable solvents include alcohols such as methanol, ethanol or isopropanol; alkylene glycols such as ethylene glycol, propylene glycol; and water. Also a combination of two or more solvents is contemplated. Preferably, the solvent is selected from alcohol, in particular methanol, ethanol and isopropanol, and water. Most preferably, the solvent is water.

In one embodiment, the reaction mixture comprises the solvent in an amount of at least 50 % by weight (wt%), based on the total weight of the reaction mixture. Preferably, the solvent is present in an amount of at least 55 wt%, more preferably at least 60 wt%, even more preferably at least 65 wt% and most preferably at least 70 wt%, and preferably at most 99 wt%, more preferably at most 95 wt%, even more preferably at most 90 wt% and most preferably at most 85 wt%, based on the total weight of the reaction mixture. The reaction mixture includes all ingredients that are necessary to convert the compound of formula (3) into the compound of formula (4), i.e. it includes the compound of formula (3), the base, the (optional) solvent and any other ingredient present.

In one aspect, this process step is conducted at a temperature of at least 20°C, preferably at least 25°C and most preferably at least 30°C, and preferably at most 100°C, more preferably at most 90°C and most preferably at most 80°C.

The invention further pertains to a process for preparing a compound of formula (3) 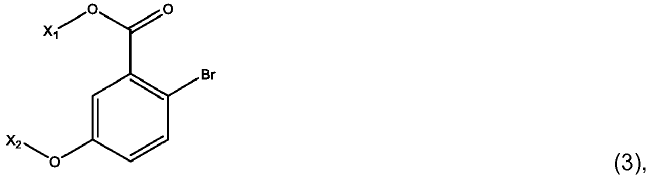 wherein X₁ and X₂ are protecting groups, and wherein at least one of the protecting groups X₁ and X₂ is hydrogen or a protecting group selected from isopropyl, tert-butyl and a protecting group comprising an oxygen,
which process comprises the steps of contacting the compound of formula (2) 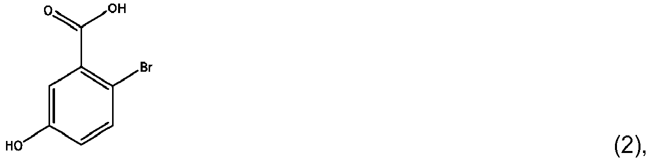 with a protecting group precursor, preferably alkoxy alkylchloride, in a solvent, and converting the compound of formula (2) into a compound of formula (3).
The protecting groups X₁ and X₂ are as defined above.

The compound of formula (2) is contacted with a protecting group precursor, preferably an alkoxy alkyl chloride, in a solvent. At least one of the protecting group precursors comprises an oxygen. Suitable protecting group precursors are precursors of protecting groups selected from alkoxy alkyl, tetrahydropyranyl (THP), ethyl vinyl ether and tert-butyl. Preferred precursors are halogenated alkoxy alkyls. Most preferably the precursor are alkoxy alkyl chlorides. Examples of alkoxy alkyl chlorides include methoxy methyl chloride, ethoxy methyl chloride, methoxy ethyl chloride and ethoxy ethyl chloride. Methoxy methyl chloride and ethoxy methyl chloride are preferred. Most preferred is methoxy methyl chloride.

In one embodiment of the invention, the molar ratio of the alkoxy alkyl chloride and the compound of formula (2) is at least 0.1, preferably at least 0.5, more preferably at least 0.8 and most preferably at least 1, and generally at most 10, preferably at most 8, more preferably at most 6 and most preferably at most 5.

A solvent is present during this process step. Examples of suitable solvents include ketones such as acetone, methyl ethyl ketone and methyl isobutyl ketone; alkyl acetates such as ethyl acetate and propyl acetate; halogenated methane such as trichloro methane and dichloro methane. Also a combination of two or more solvents is contemplated. Preferred solvents are selected from the group consisting of acetone, ethyl acetate and dichloro methane. The most preferred solvent is acetone.

In one embodiment, the reaction mixture comprises the solvent in an amount of at least 50 % by weight (wt%), based on the total weight of the reaction mixture. Preferably, the solvent is present in an amount of at least 55 wt%, more preferably at least 60 wt%, even more preferably at least 65 wt% and most preferably at least 70 wt%, and preferably at most 99 wt%, more preferably at most 95 wt%, even more preferably at most 90 wt% and most preferably at most 85 wt%, based on the total weight of the reaction mixture. The reaction mixture includes all ingredients that are necessary to convert the compound of formula (3) into the compound of formula (4), i.e. it includes the compound of formula (3), the protecting group precursor, the (optional) solvent and any other ingredient present.

In one aspect, this process step is conducted at a temperature of at least 0°C, preferably at least 10°C and most preferably at least 20°C, and preferably at most 100°C, more preferably at most 90°C and most preferably at most 80°C.

The invention further pertains to a compound of formula (3) 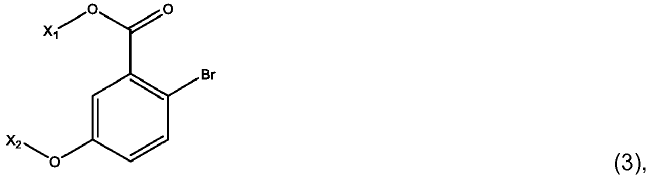 wherein X₁ and X₂ are protecting groups, and wherein at least one of the protecting groups X₁ and X₂ is hydrogen or a protecting group selected from isopropyl, tert-butyl and a protecting group comprising an oxygen.
The protecting groups X₁ and X₂ are independently chosen as defined above.

A preferred intermediate is the compound of formula (3a) 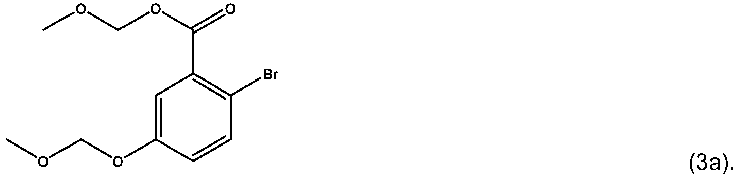 A preferred intermediate is the compound of formula (3b)

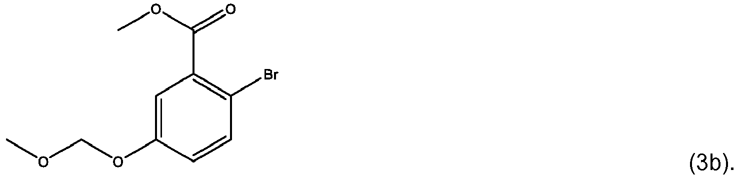

The invention further pertains to a compound of formula (4) 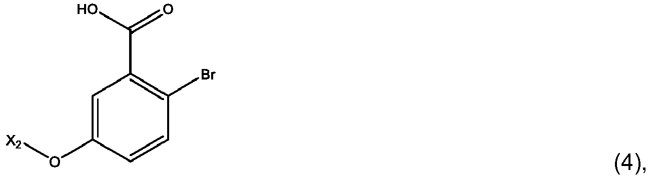 wherein X₂ is a protecting group selected from isopropyl, tert-butyl and a protecting group comprising an oxygen.
The protecting group X₂ is as defined above.

A preferred intermediate is the compound of formula (4a)

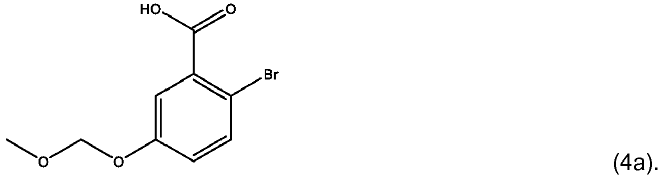

The invention further pertains to a compound of formula (5) 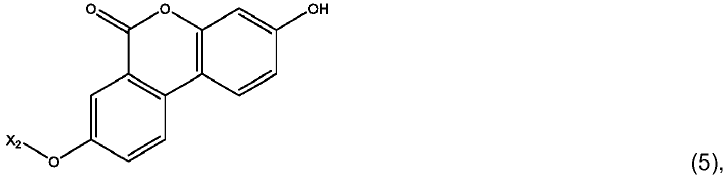 wherein X₂ is a protecting group selected from isopropyl, tert-butyl and a protecting group comprising an oxygen.
The protecting group X₂ is as defined above.

A preferred intermediate is the compound of formula (5a)

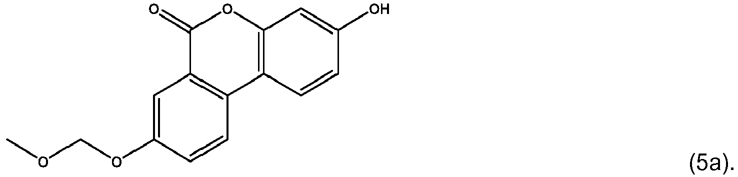

In a further embodiment of the invention, the compound of formula (3) can be prepared by contacting a compound of formula (6) 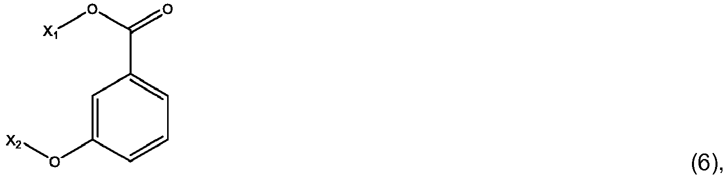 wherein at least one of the protecting groups X₁ and X₂ is hydrogen or a protecting group selected from isopropyl, tert-butyl and a protecting group comprising an oxygen, with a bromine source, preferably an organic or inorganic bromine source, more preferably bromine, to form the compound of formula (3). The compound of formula (6) can be formed by contacting 3-hydroxy benzoic acid and at least one of the protecting group precursors to form compound (6). The advantage of this route is that the overall costs of the raw materials will be lower compared to the process starting from the brominated compound of formula (2) or (3). Moreover, the regioselectivity of this process is generally high. Specific examples of compounds of formula (6) include compounds wherein X₁ and X₂ are both methoxy methyl, compounds wherein X₁ and X₂ are both ethoxy methyl, compounds wherein X₁ and X₂ are both ethyl vinyl ether, compounds wherein X₁ is H and X₂ is methoxy methyl and compounds wherein X₁ is H and X₂ is tetrahydropyranyl (THP).

The subsequently obtained compound of formula (3) can be converted to the urolithin as described above.

The various amounts and reactants as well as the conditions can be chosen as described above.

It is noted that the process of the invention can also be applied to prepare other urolithins such as urolithin B, urolithin C and urolithin D, and any derivative of the urolithins.

The invention is exemplified in the following Examples.

### Examples

### Example 1: preparing compound (3) from compound (2) and alkoxy methylchloride

20.0 g of 2-bromo-5-hydroxybenzoic acid (92.2 mmol, 1.0eq.) is charged in a flask to which 160 ml acetone is added. Subsequently, 38.2 g K₂CO₃ (277 mmol, 3.0 eq) is added resulting in a white suspension. Then methoxy methyl chloride (2.2 eq) solution in ethyl acetate is added to the reaction mixture during a period of 6 hours at room temperature.

Subsequently, 160 ml water is added to the reaction mixture rendering a bi-phasic mixture. To this mixture 80 ml of ethyl acetate is added. The water layer is removed. The water layer is subsequently washed with 50 ml ethyl acetate. The organic solvent is evaporated to dryness in vacuum at 48°C. Subsequently, 200 ml ethyl acetate was added to the residue and the solvent is evaporated to dryness. 26.8 g of methoxymethyl-2-bromo-5-(methoxymethyl) benzoate (compound according to formula (3)) was formed. The chemical structure of the resulting yellowish oil was confirmed using ¹H NMR. ¹H NMR (400 MHz, CDCl3) δ ppm 7.56 (d, J = 8.8 Hz, 1 H), 7.53 (d, J = 3.0 Hz, 1 H), 7.06 (dd, J = 8.8, 3.0 Hz, 1 H), 5.49 (s, 2 H), 5.19 (s, 2H), 3.59 (s, 3H), 3.48 (s, 3H).

### Example 2: preparing compound (4) from compound (3)

To 26.8 g of methoxymethyl-2-bromo-5-(methoxymethyl)benzoate, 84 ml of water is added resulting into an emulsion, to which 8.4 g of a 50% aqueous sodium hydroxide solution. The emulsion is stirred at 55°C for about 2.5 hours, thereby forming a clear solution. HPLC confirmed that the conversion exceeded 99%.

The solution is mixed with 135 ml MTBE, after which the temperature of the solution is reduced to 10-15°C. HCl solution is added to adjust the pH to 2.5. The layers are subsequently separated. The aqueous phase was extracted with 67 ml MTBE, and the organic layers were combined. The organic layer was washed with 50 ml water. The MTBE is evaporated at 45°C under vacuum until about 35 ml was obtained, and a solid precipitates. To the resulting suspension 250 ml heptane was added, after which the mixture was evaporated to about 150 ml at 45°C in vacuum. The resulting mixture was stirred overnight at ambient temperature. The subsequent suspension was filtered. The resulting solids are washed with 30 ml heptane, and the product was dried at 45°C under vacuum. 21.6 g of 2-bromo-5-(methoxymethyl) benzoic acid (compound according to formula (4)) is formed, which is corroborated by ¹H NMR. ¹H NMR (400 MHz, DMSO-d6) δ ppm 7.61 (d, J = 8.8 Hz, 1 H), 7.37 (d, J = 2.9 Hz, 1 H), 7.11 (dd, J = 8.8, 2.9 Hz, 1 H), 5.23 (s, 2 H), 3.38 (s, 3H).

### Example 3: preparing compound (5) from compound (4)

20.0 g of 2-bromo-5-(methoxymethyl) benzoic acid is charged under a nitrogen atmosphere, to which 17.0 g resorcinol was added. Subsequently, 130 ml water was added and a suspension was formed. 13.5 g of an aqueous sodium hydroxide (50%) was added to the reaction mixture, thereby eventually forming a clear yellow solution. 19 mg copper(II) sulfate was added. The mixture was then heated to 75°C, and stirred for about 4.5 hours. The mixture is subsequently cooled to ambient temperature, and the slurry is stirred for another 2 hours. The slurry is subsequently filtered and rinsed with 60 ml water. The product is subsequently dried at 50°C under vacuum. 13.7 g of 3-hydroxy-8-(methoxymethyl)-6H-benzo(c)chromen-6-one (compound according to formula (5)) was obtained. The chemical structure was confirmed using ¹H NMR. ¹H NMR (400 MHz, DMSO-d6) δ ppm 8.16 (d, J = 8.9 Hz, 1 H), 8.04 (d, J = 8.8 Hz, 1 H), 7.72 (d, J = 2.6 Hz, 1 H), 7.52 (dd, J = 8.8, 2.7 Hz, 1 H), 6.82 (dd, J = 8.7, 2.3 Hz, 1 H), 6.73 (d, J = 2.3 Hz, 1 H), 5.31 (s, 2 H), 3.42 (s, 3H).

### Example 4: preparing compound (1) from compound (5)

13.5 g of 3-hydroxy-8-(methoxymethyl)-6H-benzo(c)chromen-6-one was charged to a flask and 270 ml of an aqueous acetic acid solution. Add 27 ml water to the mixture, and stir the mixture at 100°C for about 5 hours. The mixture was subsequently cooled to room temperature, and the suspension was filtered. The product was washed with 10 ml aqueous acetic acid solution. The resulting product is washed with 30 ml water and 20 ml methanol. Then the washed product is dried at 50°C under vacuum. 11.0 g of a yellowish solid was obtained. The solid was confirmed with ¹H NMR to be 3,8-dihydroxy-6H-benzo(c)chromen-6-one (compound according to formula (1)). ¹H NMR (400 MHz, DMSO-d6) δ ppm 8.10 (d, J = 8.8 Hz, 1 H), 8.01 (d, J = 8.8 Hz, 1 H), 7.52 (d, J = 2.6 Hz, 1 H), 7.32 (dd, J = 8.7, 2.6 Hz, 1 H), 6.81 (dd, J = 8.7, 2.2 Hz, 1 H), 6.73 (d, J = 2.2 Hz, 1 H).

## Claims

1. A process for preparing a compound of formula (1) 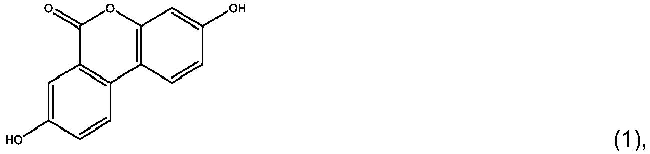 which process comprises the steps of:
a) contacting a compound of formula (2) 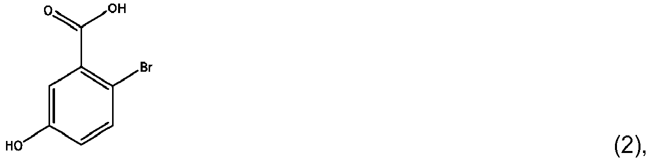 with a protecting group precursor in a solvent, and converting the compound of formula (2) into a compound of formula (3) 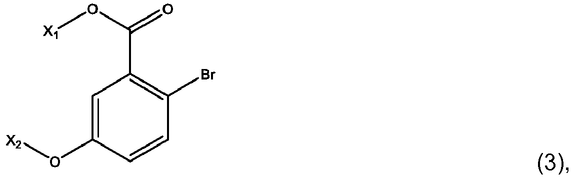
b) followed by contacting the compound of formula (3) with a base to give a compound of formula (4) 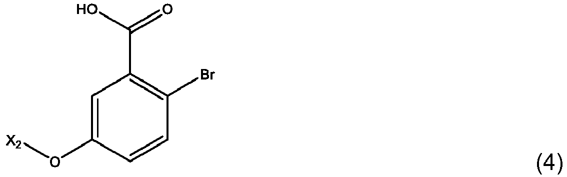
c) followed by contacting the compound of formula (4) with resorcinol in the presence of a base to give a compound of formula (5) 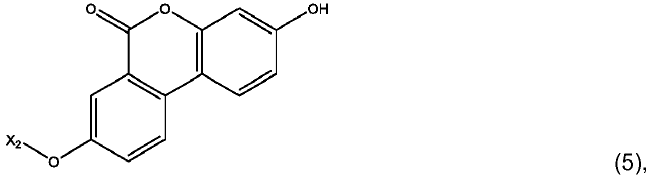 and
d) followed by contacting the compound of formula (5) with an acid to give the compound of formula (1),
and wherein at least one of the protecting groups X₁ and X₂ is hydrogen or a protecting group selected from isopropyl, tert-butyl and a protecting group comprising an oxygen.

2. A compound of formula (3) 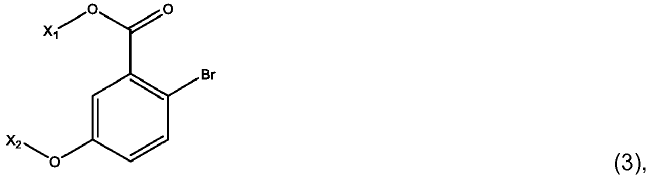 wherein X₁ and X₂ are protecting groups, and wherein at least one of the protecting groups X₁ and X₂ is hydrogen or a protecting group selected from isopropyl, tert-butyl and a protecting group comprising an oxygen.

3. A compound of formula (4) 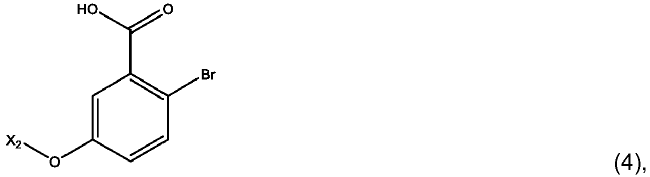 wherein X₂ is a protecting group selected from isopropyl, tert-butyl and a protecting group comprising an oxygen.

4. The compound of formula (5) 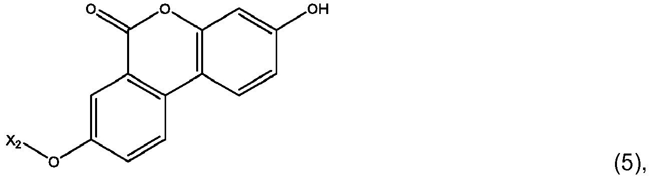 wherein X₂ is a protecting group selected from isopropyl, tert-butyl and a protecting group comprising an oxygen.

5. A process for preparing a compound of formula (1) 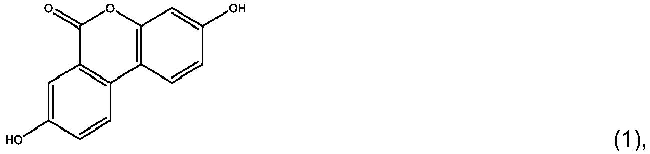 which process comprises the steps of contacting the compound of formula (5) 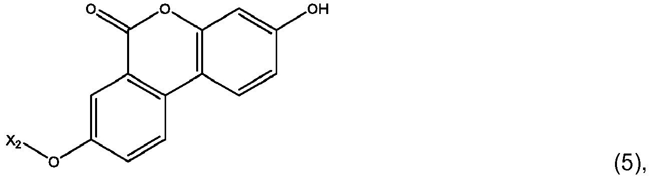 wherein X₂ is hydrogen or a protecting group selected from isopropyl, tert-butyl and a protecting group comprising an oxygen;
with an acid to give the compound of formula (1).

6. A process for preparing a compound of formula (5) 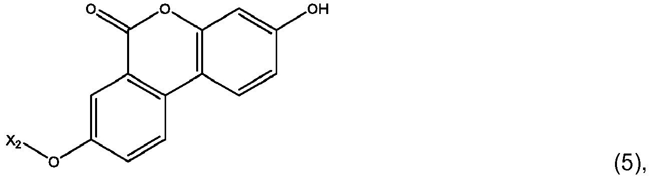 wherein X₂ is hydrogen or a protecting group selected from isopropyl, tert-butyl and a protecting group comprising an oxygen;
which process comprises the steps of contacting the compound of formula (4) 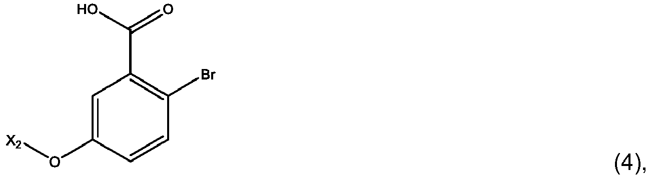 wherein X₂ is hydrogen or a protecting group selected from isopropyl, tert-butyl and a protecting group comprising an oxygen,
with resorcinol in the presence of a base to give the compound of formula (5).

7. A process for preparing a compound of formula (4) 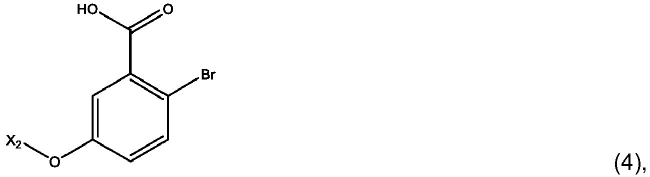 wherein X₂ is hydrogen or a protecting group selected from isopropyl, tert-butyl and a protecting group comprising an oxygen,
which process comprises the steps of contacting the compound of formula (3) 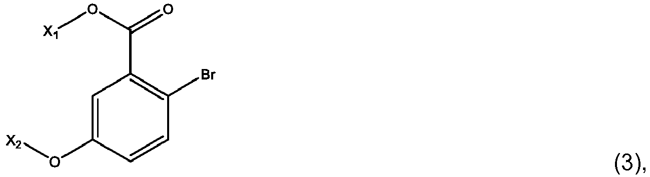 wherein X₁ and X₂ are protecting groups, and wherein at least one of the protecting groups X₁ and X₂ is hydrogen or a protecting group selected from isopropyl, tert-butyl and a protecting group comprising an oxygen,
with a base to give the compound of formula (4).

8. A process for preparing a compound of formula (3) 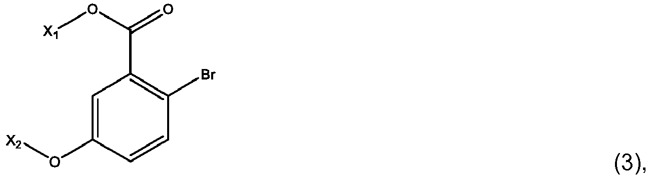 wherein X₁ and X₂ are protecting groups, and wherein at least one of the protecting groups X₁ and X₂ is hydrogen or a protecting group selected from isopropyl, tert-butyl and a protecting group comprising an oxygen,
which process comprises the steps of contacting the compound of formula (2) 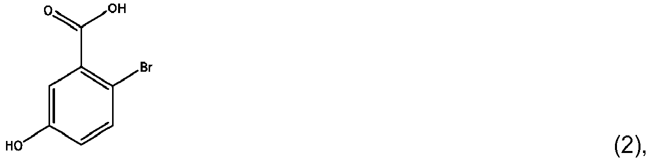 with a protecting group precursor in a solvent, and converting the compound of formula (2) into a compound of formula (3).
